# EUROPEAN PATENT APPLICATION

(11) **EP 2 036 500 A1**
(43) Date of publication of application: **18.03.2009**
(21) Application number: 08013565.0
(22) Date of filing: 28.07.2008
(51) Int. Cl.: A61B 8/12, A61B 8/08, G01S 15/89, G01N 29/26

(54) **Ultrasound diagnostic apparatus**

(30) Priority: 11.09.2007 JP 2007235800
(71) Applicant: Olympus Medical Systems Corporation, Tokyo 151-0072 (JP)
(72) Inventor: Hibi, Yasushi, Shibuya-ku, Tokyo 151-0072 (JP)
(74) Representative: von Hellfeld, Axel

(57) **Abstract**

An ultrasound diagnostic apparatus (1) of the invention includes: a plurality of transducer groups (31,32) provided to a distal end portion (20a) of a probe, for scanning in planes that are orthogonal to each other; display means (70) including a display screen (71) capable of simultaneously displaying a plurality of ultrasound images (31E,32E) obtained by the scanning by the respective transducer groups; and image synthesis means (51) for displaying, on the display screen (71), selected one of a plurality of aspects of display wherein the positional relation of the plurality of transducer groups (31,32) on the probe and the positional relation of the plurality of ultrasound images (31E,32E) on the display screen are the same. The ultrasound diagnostic apparatus (1) can thus provide simultaneous display of a plurality of ultrasound images and good operability.

## Description

### BACKGROUND OF THE INVENTION

### 1. Field of the Invention

The present invention relates to an ultrasound diagnostic apparatus, and in particular to an ultrasound diagnostic apparatus including a plurality of transducer groups provided to a distal end portion of a probe, for scanning in planes that are orthogonal to each other; and displaying means for simultaneously displaying a plurality of ultrasound images.

### 2. Description of Related Art

Recent years have seen wide use of diagnostic methods where an ultrasound endoscope is inserted into a living body to obtain an optical image from which to find a lesion part in the body, and ultrasound is irradiated thereto to use its reflection wave to create an ultrasound tomographic image of the lesion part to conduct diagnosis. Also, methods have been implemented that an operator, while visually checking a guiding ultrasound tomographic image, punctures a puncture needle into a subject to aspirate cells to serve for making a definite diagnosis.

The definite diagnosis requires precisely collecting the tissues. The collecting methods include puncturing a needle into a tumor under the guidance of a B-mode ultrasound image to surely collect the tumor. In this collecting method, the radial scan capable of scanning the entire inner lumen is suitable for diagnosing an inner lumen lesion part such as in a living body. The operator first diagnoses the lesion part using an intra-body cavity ultrasound endoscope for radial scan. After confirming the lesion part position, the operator further inserts into the patient an intra-body cavity ultrasound endoscope for convex scan which facilitates confirming the biopsy needle position to again find the lesion part as required to make a definite diagnosis. Thus, the patient is required to swallow the intra-body cavity ultrasound endoscopes twice, subjected to pain.

Japanese unexamined patent publication No. 8-56948 discloses a biplane ultrasound diagnostic apparatus wherein a transducer for radial scan and a transducer group for linear scan are closely arranged on a probe so that the scanning directions intersect with each other. This ultrasound diagnostic apparatus permits observation of section images in different directions with a single scope. The TV monitor can simultaneously display a radial scan based image and a linear scan based image.

Further, Japanese Unexamined Patent Publication No. 2002-177278 discloses an ultrasound diagnostic apparatus using a probe including three transducers, and adjacently displaying three ultrasound images scanned by the three transducers on a single TV monitor.

Furthermore, Japanese unexamined patent publication No. 11-318904 discloses an ultrasound diagnostic apparatus that transmits and receives ultrasound to and from a living body to perform a three-dimensional scan to obtain echo data of a three-dimensional region, which is used to display an intra-living body ultrasound image on an ultrasound image device. When focusing on only one of radial and linear tomographic images on the display screen, only the one image is enlargedly displayed to provide efficient use of a limited screen space of the image device and an easy-to-view display.

When adjacently displaying a plurality of ultrasound images on the single display screen, it is necessary for easy understanding for the operator to show, on the display screen, information indicating the relationship, etc. between the ultrasound images and their respective scanning positions.

However, in the biplane ultrasound diagnostic apparatus disclosed in Japanese unexamined patent publication No. 8-56948, even though the TV monitor can simultaneously display the radial scan based and linear scan based images, the positional relation between the displayed images is fixed. That is, the left half of the TV monitor always displays the radial scan based image and the right half the linear scan based image. With this fixed display method, an operator not used to the display method has difficulty to understand the positional relation of the transducers, which results in bad operability of the ultrasound diagnostic apparatus. The fixed display method is also inconvenient when the operator operates the apparatus while referring to an image in a different display method by another ultrasound diagnostic apparatus.

Moreover, in the ultrasound diagnostic apparatus disclosed in Japanese Unexamined Patent Publication No. 2002-177278, the three ultrasound images scanned by the three transducers A, B, C are merely displayed with the symbols A, B, C on the single TV monitor. The operator is thus required to operate the apparatus, paying attention to the relationship between the respective scanning positions of the transducers and the displayed ultrasound images.

An object of the present invention is to provide an ultrasound diagnostic apparatus with good operability for simultaneously displaying a plurality of ultrasound images.

### SUMMARY OF THE INVENTION

An ultrasound diagnostic apparatus of the invention includes: a plurality of transducer groups provided to a distal end portion of a probe, for scanning in planes that are orthogonal to each other; display means including a display screen capable of simultaneously displaying a plurality of ultrasound images obtained by the scanning by the respective transducer groups; and image synthesis means for displaying, on the display screen, selected one of a plurality of aspects of display wherein the positional relation of the plurality of transducer groups on the probe and the positional relation of the plurality of ultrasound images on the display screen are the same.

### BRIEF DESCRIPTION OF THE DRAWINGS

FIG 1 is a configuration diagram of an ultrasound diagnostic apparatus according to a first embodiment of the present invention.
FIG 2 is perspective view of a distal end portion of a probe of an ultrasound endoscope according to the first embodiment of the present invention.
FIG 3A is a front view of the distal end portion of the probe according to the first embodiment of the present invention.
FIG 3B is a side view of the distal end portion of the probe according to the first embodiment of the present invention.
FIG. 4 is a perspective view of the distal end portion of the probe according to the first embodiment of the present invention.
FIG 5A is a side view of the distal end portion of the probe according to the first embodiment of the present invention as observed from a viewpoint E1.
FIG 5B is a side view of the distal end portion of the probe according to the first embodiment of the present invention as observed from a viewpoint E2.
FIG 5C is a side view of the distal end portion of the probe according to the first embodiment of the present invention as observed from a viewpoint E3.
FIG 5D is a side view of the distal end portion of the probe according to the first embodiment of the present invention as observed from a viewpoint E4.
FIG 6A is a view showing an exemplary aspect of display of display means 70 according to the first embodiment of the present invention.
FIG 6B is a view showing an exemplary aspect of display of the display means 70 according to the first embodiment of the present invention.
FIG 6C is a view showing an exemplary aspect of display of the display means 70 according to the first embodiment of the present invention.
FIG 6D is a view showing an exemplary aspect of display of the display means 70 according to the first embodiment of the present invention.
FIG. 7A is a view showing a positional relation of scanning planes of an ultrasound diagnostic apparatus including a plurality of transducer groups for scanning in planes that are orthogonal to each other.
FIG 7B is a view showing a positional relation of scanning planes of the ultrasound diagnostic apparatus including a plurality of transducer groups for scanning in planes that are orthogonal to each other.
FIG 7C is a view showing a positional relation of scanning planes of the ultrasound diagnostic apparatus including a plurality of transducer groups for scanning in planes that are orthogonal to each other.
FIG. 8A is a view showing a positional relation of scanning planes of the ultrasound diagnostic apparatus including a plurality of transducer groups for scanning in planes that are orthogonal to each other.
FIG 8B is a view showing a positional relation of scanning planes of the ultrasound diagnostic apparatus including a plurality of transducer groups for scanning in planes that are orthogonal to each other.
FIG 8C is a view showing a positional relation of scanning planes of the ultrasound diagnostic apparatus including a plurality of transducer groups for scanning in planes that are orthogonal to each other.
FIG 9A is a view showing a positional relation of scanning planes of the ultrasound diagnostic apparatus including a plurality of transducer groups for scanning in planes that are orthogonal to each other.
FIG 9B is a view showing a positional relation of scanning planes of the ultrasound diagnostic apparatus including a plurality of transducer groups for scanning in planes that are orthogonal to each other.
FIG 9C is a view showing a positional relation of scanning planes of the ultrasound diagnostic apparatus including a plurality of transducer groups for scanning in planes that are orthogonal to each other.
FIG 10 is a view showing an aspect of display of a display device in the ultrasound diagnostic apparatus including a plurality of transducer groups for scanning in planes that are orthogonal to each other.
FIG 11 is a view showing an aspect of display of a display device in the ultrasound diagnostic apparatus including a plurality of transducer groups for scanning in planes that are orthogonal to each other.
FIG 12 is a view showing an aspect of display of a display device in the ultrasound diagnostic apparatus including a plurality of transducer groups for scanning in planes that are orthogonal to each other.
FIG 13 is a view showing an aspect of display of an ultrasound diagnostic apparatus of a second embodiment of the present invention.
FIG 14 is a view showing an aspect of display of the ultrasound diagnostic apparatus of the second embodiment of the present invention.
FIG 15 is a view showing an aspect of display of an ultrasound diagnostic apparatus of a third embodiment of the present invention.
FIG 16 is a view showing an aspect of display of the ultrasound diagnostic apparatus of the third embodiment of the present invention.
FIG 17 is a view showing an aspect of display including auxiliary calibration marks.
FIG 18 is a view showing an aspect of display including auxiliary calibration marks.
FIG 19 is a view showing an aspect of display displaying concentric circle-shaped auxiliary calibration marks centering a puncture point of a biopsy needle.
FIG 20 is a view showing an aspect of display displaying concentric circle-shaped auxiliary calibration marks centering a puncture point of a biopsy needle.
FIG 21 is a view showing an aspect of display including auxiliary calibration marks.
FIG 22 is a view showing an aspect of display including auxiliary calibration marks.

### DETAILED DESCRIPTION OF PREFERRED EMBODIMENTS

Embodiments of the present invention are described below with reference to the drawings.

### <First Embodiment>

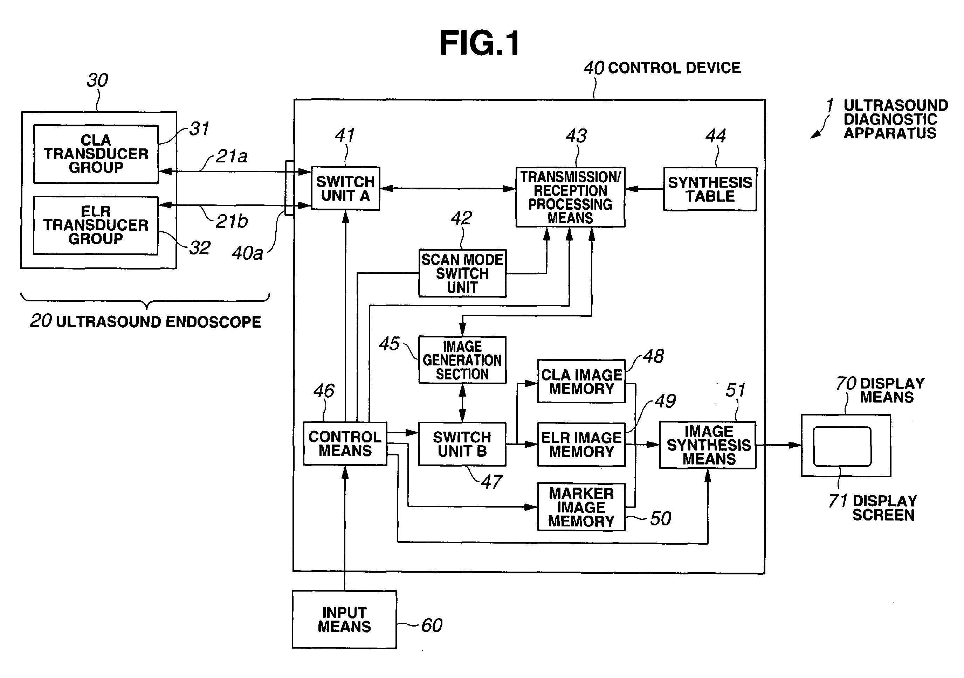

FIG 1 is a configuration diagram of an ultrasound diagnostic apparatus 1 according to a first embodiment of the present invention. The ultrasound diagnostic apparatus 1 of the present embodiment includes an ultrasound endoscope 20, a control device 40, input means 60 which is connected to and operates the control device 40, and display means 70 which is likewise connected to the control device 40 and displays image information obtained by the control device 40.

The ultrasound endoscope 20 is inserted into a body cavity, etc. to transmit an ultrasound beam toward an observation target region and receives a reflection wave reflected from a border of acoustic impedance of the observation target region, thus obtaining an echo signal. The control device 40 is connected to the ultrasound endoscope 20 by cables 21a and 22b via a connector 40a, to control transmission and reception by the ultrasound endoscope 20.

The ultrasound endoscope 20 includes an elongate probe to be inserted into a body cavity, etc.; and transducer groups 30 including two transducer groups of a CLA (Curved Linear Array) transducer group 31 and an ELR (ELectrical Radial) transducer group 32, provided to a distal end portion of the probe, for transmitting and receiving ultrasound beam. Note that the CLA transducer group is the same as a convex transducer group and the ELR transducer group is a transducer group that electronically performs radial scan. Note also that in mechanical radial scan which mechanically performs radial scan, a single transducer is used in some cases. In the present embodiment, such a single transducer for mechanical radial scan is also included in a transducer group.

The control device 40 includes a switch unit A 41 for selecting and switching to one of the transducer groups 31 and 32; a scan mode switch unit 42 for switching between B-mode scan and Doppler scan; transmission-reception processing means 43 used for performing a scan with a transducer group selected by the switch unit A 41 in a scan mode selected by the scan mode switch unit 42 and conducting an echo signal detection, etc.; an image generation section 45 for generating display image data from data obtained by the transmission-reception processing means 43; a switch unit B47 for storing an image signal of the transducer group 31 to its image memory, i.e., a CLA image memory 48, or storing an image signal of the transducer group 32 to its image memory, i.e., an ELR image memory 49; a marker image memory 50 for displaying a marker for image selection; image synthesis means 51 for synthesizing images; and control means 46 for controlling the entire ultrasound diagnostic apparatus. The switch unit A 41 may employ a multiplexer (MUX).

A CLA image stored in the CLA image memory 48, an ELR image stored in the ELR image memory 49, and an image from the marker image memory 50 are synthesized by the image synthesis means 51 into an aspect of display according to an instruction from the input means 60 via the control means 46. The synthesized image is displayed on a display screen 71 of the display means 70.

The control device 40 further includes a synthesis table 44. The transmission-reception processing means 43 gives time differences to drive signals to be sent to the respective transducer elements (hereinafter "transducers") configuring each of the transducer groups, in accordance with control data stored in the synthesis table 44. As a result, ultrasounds having phase differences according to the time differences of the drive signals are transmitted from the respective transducers. By wavefront synthesis of the transmitted ultrasounds having phase differences, a single ultrasound ray is formed along a sound ray in a predetermined orientation. Reflected returning echo signals of the transmitted ultrasounds are received by the respective transducers that transmitted the ultrasounds, and synthesized by the transmission-reception processing means 43 also according to the control data stored in the synthesis table 44 into a single-frame signal as frame data for image generation.

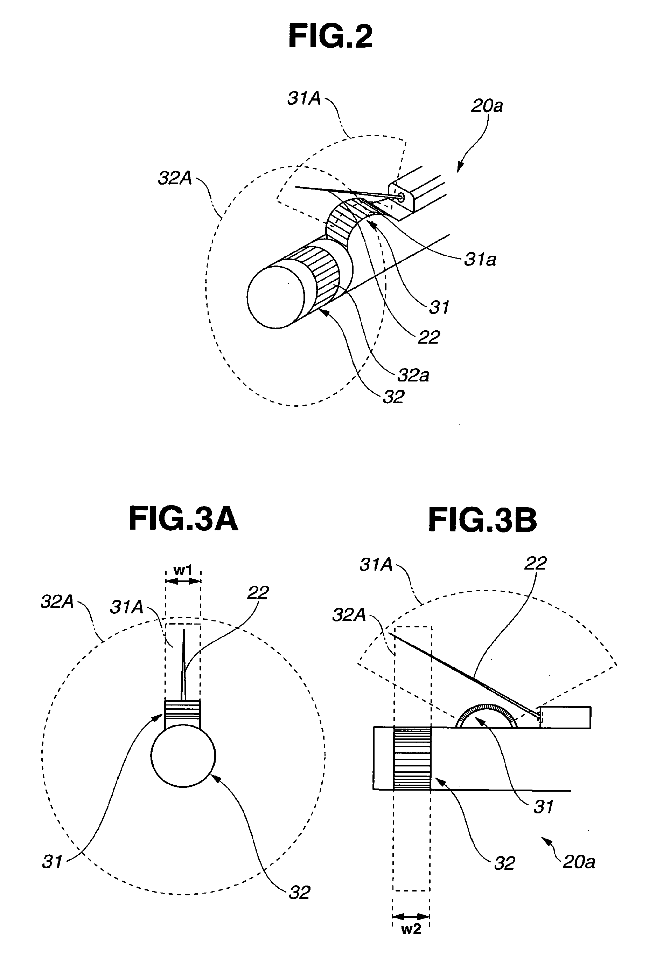

FIG 2 is a perspective view of a distal end portion 20a of the probe of the present embodiment. The CLA transducer group 31 conducts a scan in a sector-shape in a plane that is parallel to a probe shaft, as shown by a CLA transducer group scanning plane 31 A. The CLA transducer group 31 includes transducers 31 a that are arranged in an arc shape. In contrast, the ELR transducer group 32 conducts a scan in a circular shape in a plane that is perpendicular to the probe shaft, as shown by an ELR transducer group scanning plane 32A. The ELR transducer group 32 includes transducers 32a that are arranged in a circumferential shape. A biopsy needle 22 for collecting a cell specimen is punctured along the CLA transducer group scanning plane 31 A.

FIG 3A is a front view of the distal end portion 20a of the probe of the present embodiment as observed from a probe insertion direction. FIG 3B is a side view of the distal end portion 20a. As shown in FIGS. 3A and 3B, the CLA transducer group scanning plane 31 A and the ELR transducer group scanning plane 32A are orthogonal to each other. The CLA transducer group scanning plane 31 A and the ELR transducer group scanning plane 32A have depths that correspond to widths w1 and w2 of the respective transducers.

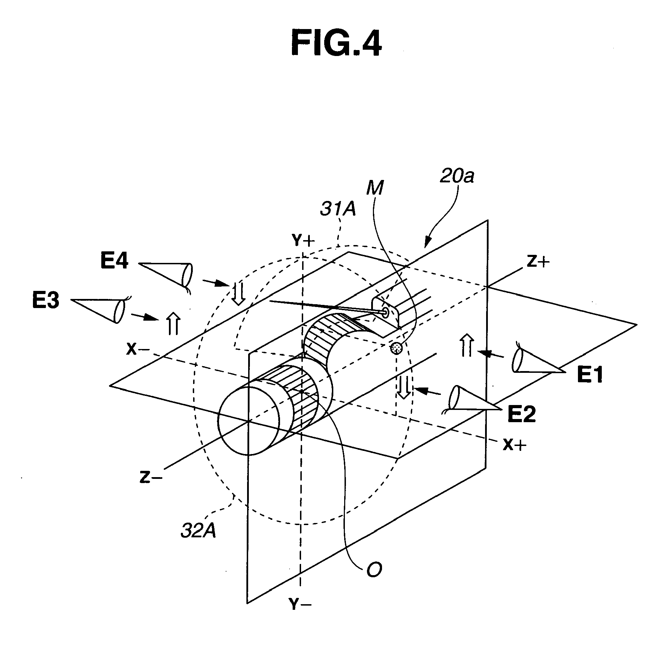

FIG 4 is a perspective view of the distal end portion 20a of the probe of the present embodiment for illustrating observational viewpoints of positional relations. In FIG 4, an intersection between the probe shaft center and the ELR transducer group scanning plane 32A is set as an origin O, a plane including the ELR transducer group scanning plane 32A as an XY plane, a plane including the CLA transducer group scanning plane 31 A as a YZ plane, and a plane orthogonal to the XY and YZ planes as an XZ plane. Furthermore, rightward, upward and depthward directions on the drawing are indicated as X+, Y+ and Z+ directions, respectively. The reference symbol M shows a part of a subject (not shown).

Using FIG 4, a positional relation between the CLA transducer group 31 and the ELR transducer group 32 is described.

Specifically, a positional relation of the transducer groups refers to at least one of: left-right relation as to which of the left and right directions of the CLA transducer group 31 the ELR transducer group 32 is positioned in on the probe; and up-down relation as to which of the up and down directions of the CLA transducer group 31 the ELR transducer group 32 is positioned in on the probe.

Because the positional relation of the transducer groups is relative, the relation varies with observing position and direction, in other words, the viewpoint. In the ultrasound diagnostic apparatus, determining a viewpoint using the transducer group scanning plane as the basis facilitates the operator to understand the positional relation of the transducer groups, leading to good operability of the ultrasound diagnostic apparatus. In other words, the positional relation of the transducer groups preferable for the operator is that of observing from a viewpoint orthogonal to selected one of the scanning planes of the plurality of transducer groups, that is, a viewpoint to laterally observe the selected scanning plane.

For example, in FIG 4, when the scanning plane 31A is selected, there are four viewpoints to observe the YZ plane from the X-axis direction. That is, a viewpoint E1 observes the distal end portion 20a of the probe from the X+ side with the Y+ side on the upside. A viewpoint E2 observes the distal end portion 20a of the probe from the X+ side with the Y- side on the upside. A viewpoint E3 observes the distal end portion 20a of the probe from the X- side with the Y+ side on the upside. A viewpoint E4 observes the distal end portion 20a of the probe from the X- side with the Y- side on the upside.

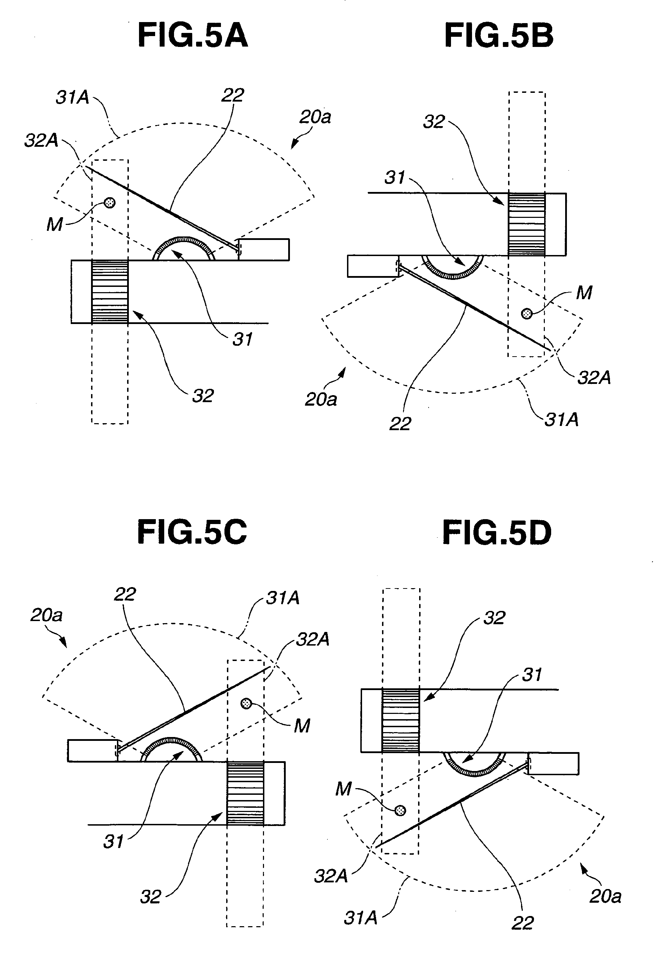

FIGS. 5A to 5D are side views of the distal end portion 20a of the probe when the distal end portion 20a of the probe shown in FIG 4 is observed from the four viewpoints. FIG 5A is a side view of the distal end portion 20a of the probe when the distal end portion 20a of the probe is observed from the viewpoint E1, FIG 5B from the viewpoint E2, FIG 5C from the viewpoint E3, and FIG 5D from the viewpoint E4.

In FIG 5A, the ELR transducer group scanning plane 32A is on the left side of the CLA transducer group scanning plane 31A. The CLA transducer group scanning plane 31 A spreads upward in a sector shape. The biopsy needle 22 is punctured from the lower right toward the upper left along the CLA transducer group scanning plane 31 A. A part M of the subject is on the upside of the ELR transducer group scanning plane 32A.

In FIG 5B, the ELR transducer group scanning plane 32A is on the right side of the CLA transducer group scanning plane 31A. The CLA transducer group scanning plane 31 A spreads downward in a sector shape. The biopsy needle 22 is punctured from the upper left toward the lower right along the CLA transducer group scanning plane 31A. The part M of the subject is on the downside of the ELR transducer group scanning plane 32A.

In FIG 5C, the ELR transducer group scanning plane 32A is on the right side of the CLA transducer group scanning plane 31 A. The CLA transducer group scanning plane 31A spreads upward in a sector shape. The biopsy needle 22 is punctured from the lower left toward the upper right along the CLA scanning plane 31A. The part M of the subject is on the upside of the ELR transducer group scanning plane 32A.

In FIG 5D, the ELR transducer group scanning plane 32A is on the left side of the CLA transducer group scanning plane 31A. The CLA transducer group scanning plane 31A spreads downward in a sector shape. The biopsy needle 22 is punctured from the upper right toward the lower left along the CLA transducer group scanning plane 31A. The part M of the subject is on the downside of the ELR transducer group scanning plane 32A.

In the ultrasound diagnostic apparatus 1 of the present embodiment, a display image for the CLA transducer group scanning plane is once stored in the CLA image memory 48, and a display image for the ELR transducer group scanning plane in the ELR image memory 48, as shown in FIG 1. The display images are then synthesized with each other by the image synthesis means 51 to be displayed on the display screen 71 of the display means 70. The image synthesis means processes each of the display images before synthesizing them. Thus, the aspect of display of the display screen, that is, the positional relation of the ultrasound images is displayed as selected one of a plurality of aspects of display according to an instruction from the input means 60.

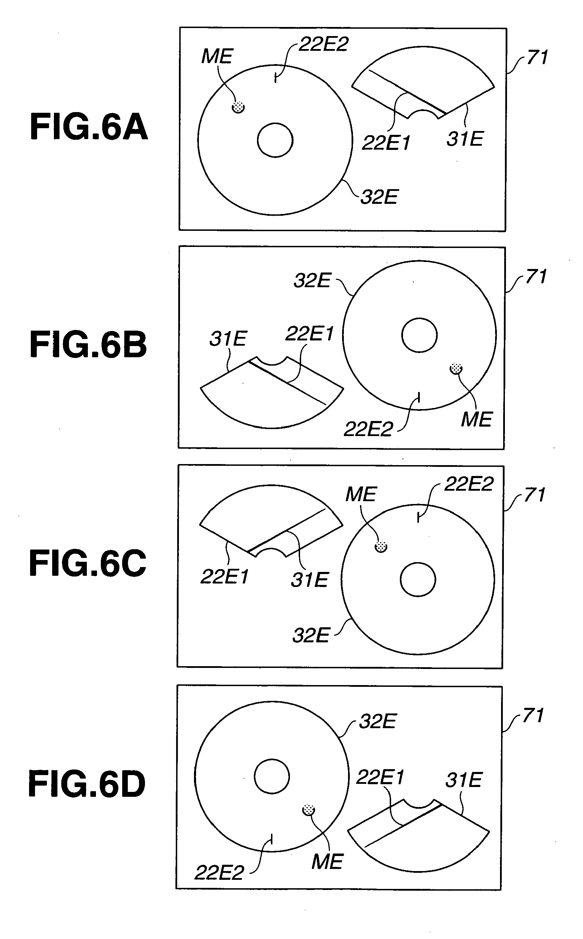

FIGS. 6A to 6D are views to illustrate exemplary aspects of display.

FIG 6A shows a display corresponding to the viewpoint E1, that is, the positional relation between the ELR transducer group scanning plane 32A and the CLA transducer group scanning plane 31 A in FIG 5A. An ELR transducer group scanning image 32E is on the left side of a CLA transducer group scanning image 31E. The CLA transducer group scanning image 31E spreads upward in a sector shape. An image of a biopsy needle 22E1 extends from the lower right to the upper left in the CLA transducer group scanning image 31E. The part M of the subject indicated by reference symbol ME is on the upside of the ELR transducer group scanning image 32E.

FIG. 6B shows a display corresponding to the viewpoint E2, that is, the positional relation between the ELR transducer group scanning plane 32A and the CLA transducer group scanning plane 31 A in FIG 5B. The ELR transducer group scanning image 32E is on the right side of the CLA transducer group scanning image 31E. The CLA transducer group scanning image 31E spreads downward in a sector shape. The image of the biopsy needle 22E1 extends from the upper left to the lower right in the CLA transducer group scanning image 31E. The part M of the subject indicated by reference symbol ME is on the downside of the ELR transducer group scanning image 32E.

FIG 6C shows a display corresponding to the viewpoint E3, that is, the positional relation between the ELR transducer group scanning plane 32A and the CLA transducer group scanning plane 31 A in FIG 5C. The ELR transducer group scanning image 32E is on the right side of the CLA transducer group scanning image 31E. The CLA transducer group scanning image 31E spreads upward in a sector shape. The image of the biopsy needle 22E1 extends from the lower left to the upper right in the CLA transducer group scanning image 31E. The part M of the subject indicated by reference symbol ME is on the upside of the ELR transducer group scanning image 32E.

FIG 6D shows a display corresponding to the viewpoint E4, that is, the positional relation between the ELR transducer group scanning plane 32A and the CLA transducer group scanning plane 31 A in FIG 5D. The ELR transducer group scanning image 32E is on the left side of the CLA transducer group scanning image 31E. The CLA transducer group scanning image 31E spreads downward in a sector shape. The biopsy needle 22E1 extends from the upper right to the lower left in the CLA transducer group scanning image 31E. The reference symbol ME that indicates the part M of the subject is on the downside of the ELR transducer group scanning image 32E.

In any of the above four aspects of display, the positional relation of the transducer groups and the positional relation of the ultrasound images are the same, i.e., the same left-right relation. Also, the up-down direction of the scanning planes of the respective transducer groups and the up-down direction of the ultrasound images are the same in positional relation.

The operator uses the input device 60 to make a selection from the above four aspects of display. The operator can select an aspect of display that is easiest to view and understand depending on a practice or examinee. The ultrasound diagnostic apparatus of the present embodiment thus provides a good operability.

Note that, in the present embodiment, the CLA transducer group 31 is configured of the plurality of transducers 31 a arranged in an arc shape, and the ELR transducer group 32 is configured of the plurality of transducers 32a arranged in a circumference shape. A radius of curvature r1 of the CLA transducer group 31 is the same as a radius of curvature r2 of the ELR transducer group 32. Thus, respective sound rays 31L and 32L are transmitted in the same direction. Furthermore, in the present embodiment, an arrangement pitch θp1 of the transducers 31 a of the CLA transducer group 31 is the same as an arrangement pitch θp2 of the transducers 32a of the ELR transducer group 32. This permits the CLA transducer group 31 and the ELR transducer group 32 to commonly use a sound ray synthesis table for storing control data of each of the transducer groups, and the transmission-reception processing means 43, which leads to a simplified configuration of the apparatus.

The above embodiment has described an ultrasound diagnostic apparatus in an example including two transducer groups, each one of the ELR transducer group and the CLA transducer group. However, the embodiment also applies to an ultrasound diagnostic apparatus including three or more transducer groups.

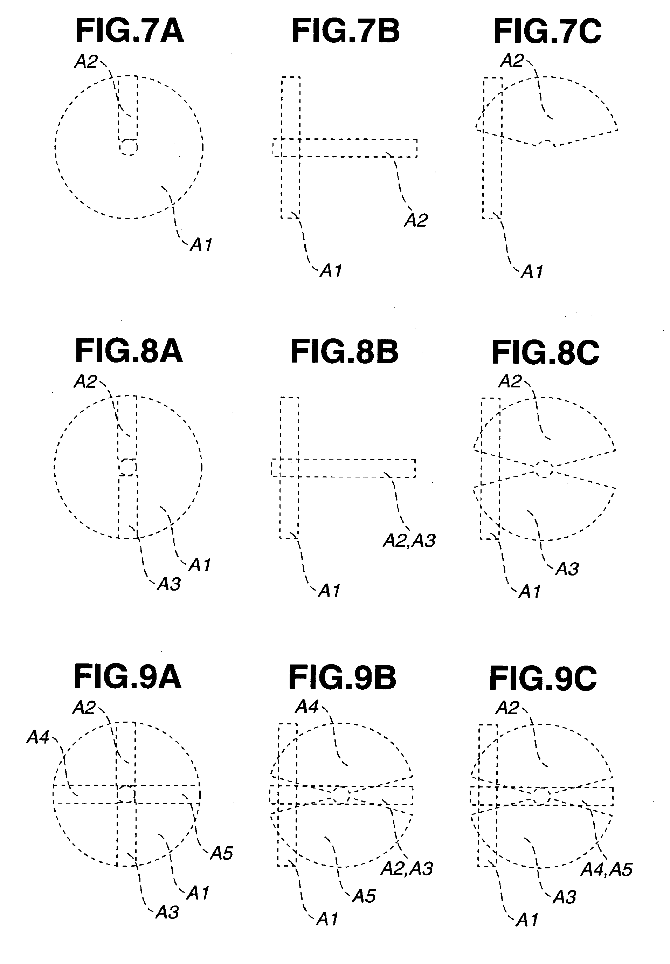

FIGS. 7A to 7C, FIGS. 8A to 8C, and FIGS. 9A to 9C are views showing scanning planes and positional relations of a plurality of transducer groups in an ultrasound diagnostic apparatus for scanning in planes that are orthogonal to each other. Using the coordinate system of FIG 4 for description, FIGS. 7A, 8A and 9A represent a viewpoint to view the XY plane from Z- direction with Y+ positioned on the upside. FIGS. 7B, 8B and 9B represent a viewpoint to view the XZ plane from Y+ direction with X- on the upside. FIGS. 7C, 8C and 9C represent a viewpoint to view the YZ plane from X+ direction with Y+ on the upside.

In other words, FIGS. 7A to 7C represent an example, same as shown in FIG 2, to scan two planes A1 and A2 by two transducer groups; each one of the ELR transducer group and the CLA transducer group. In contrast, FIGS. 8A to 8C represent an example to scan three planes A1, A2 and A3 using three transducer groups; one ELR transducer group and two CLA transducer groups. FIGS. 9A to 9C represent an example to scan five planes A1, A2, A3, A4 and A5 using five transducer groups; one ELR transducer group and four CLA transducer groups. Note that an embodiment including three or more transducer groups has the same basic configuration as in the embodiment including two transducer groups.

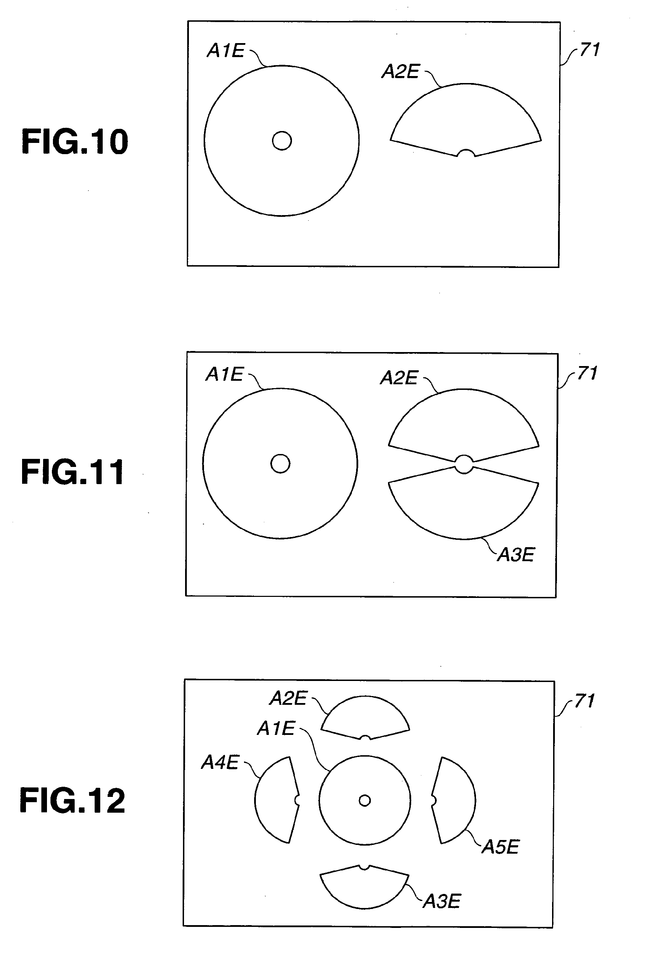

FIGS. 10 to 12 show aspects of display of a display device in the ultrasound diagnostic apparatus including a plurality of transducer groups for scanning in the planes orthogonal to each other. FIG 10 corresponds to an ultrasound diagnostic apparatus for performing the scanning of FIGS. 7A to 7C. FIG 11 corresponds to an ultrasound diagnostic apparatus for performing the scanning of FIGS. 8A to 8C. FIG 12 corresponds to an ultrasound diagnostic apparatus for performing the scanning of FIGS. 9A to 9C. Reference symbols A1E, A2E, A3E, A4E, A5E respectively represent ultrasound images of the scanning planes A1, A2, A3, A4, A5 in FIGS. 7A to 9C.

Note that in FIG 12, the observation viewpoint is different from those described heretofore. That is, in the aspect of display of FIG. 12, the observation viewpoint selected to determine the positional relation of the transducer groups is a viewpoint that is orthogonal to the ELR transducer group scanning plane A1 of the ELR transducer group as a transducer group selected. To express in the coordinate system of FIG 4, the selected observation viewpoint views the XY plane from the Z-side with the Y+ side on the upside.

The aspect of display of FIG 12 may of course be represented in the positional relation from the above described viewpoint E1 in FIG. 4. In this case, the ultrasound images A4E and A5E may not be displayed. Likewise, when the aspect of display of FIG 12 is represented in a positional relation with a viewpoint to observe the XZ plane from the Y+ side with the X- side on the upside in FIG 4, the ultrasound images A4E and A5E are displayed, but the ultrasound images A2E and A3E may not be displayed.

Thus, referring to FIG 4, it is seen that in the ultrasound diagnostic apparatus of FIGS. 9A to 9C that scans the five planes A1, A2, A3, A4 and A5, the four viewpoints orthogonal to the XY plane and the four viewpoints orthogonal to the XZ plane also represent positional relations to provide aspects of display providing a good operability for some operators.

Referring to FIG 4, it is also seen that the probe may include on the Z+ side of the CLA transducer group 31 additional one or more ELR transducer groups 32 to simultaneously display a plurality of ultrasound images scanned by the respective transducer groups, to display selected one of a plurality of aspects of display having the same positional relation between the transducer groups and the ultrasound images. Even such an ultrasound diagnostic apparatus including many transducer groups has the same basic configuration as in the embodiment including two transducer groups.

### <Second Embodiment>

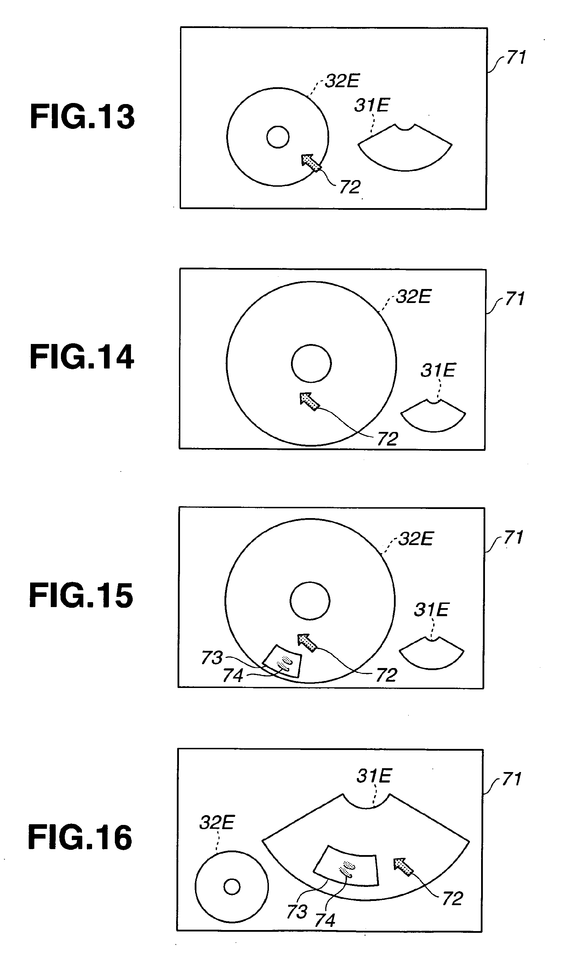

FIGS. 13 and 14 are views each showing an aspect of display of an ultrasound diagnostic apparatus of the second embodiment of the present invention. The basic configuration of the ultrasound diagnostic apparatus of the present embodiment is the same as in the first embodiment shown in FIG 1.

In the ultrasound diagnostic apparatus of the present embodiment, the operator uses the input means 60 to select the ELR transducer group scanning image 32E as one ultrasound image with a marker 72 on the display screen 71 of the display means 70, as shown in FIG 13. Specifically, the marker 72 is formed in the marker image memory 50 and is displayed on the display screen 71 of the display means 70, synthesized by the image synthesis means 51 at a position in the ultrasound image according to an input signal from the input means 60 which is processed by the control means 46.

As shown in FIG 14, the selected ELR transducer group scanning image 32E is displayed on the display screen 71, processed by the image synthesis means 51 to be displayed relatively larger than the CLA transducer group scanning image 31E as unselected another ultrasound image. Here, the word "relatively" does not mean the absolute largeness or smallness, but the proportion of size between the selected ELR transducer group scanning image 32E and the unselected CLA transducer group scanning image 31E. In the aspect of display of FIG 14, the selected ELR transducer group scanning image 32E is displayed large and the unselected CLA transducer group scanning image 31E small as compared to the pre-selection aspect of display of FIG. 13. Of course, only the selected ELR transducer group scanning image 32E may be displayed in an increased size without changing the size of the unselected CLA transducer group scanning image 31E. It is noted that the selection mechanism with which the operator selects one ultrasound image is not limited to the marker, but a known selection mechanism such as keyboard input may be used. Also, an enlarging ratio can be instructed from the input means 60 so that the image synthesis means 51 therewith processes each image.

The ultrasound diagnostic apparatus of the present embodiment thus provides enlarged and reduced displays of the relative display sizes of a plurality of simultaneously displayed ultrasound images, thereby allowing effective use of the limited space of the display screen 71 and operator selectability of an easy-to-view aspect of display to be displayed on the display screen 71.

### <Third Embodiment>

FIGS. 15 and 16 are each a view showing an aspect of display of an ultrasound diagnostic apparatus of a third embodiment of the present invention. The basic configuration of the ultrasound diagnostic apparatus of the present embodiment is the same as in the first embodiment shown in FIG 1. Note that a B-mode scan is normally performed in the ultrasound diagnostic apparatus and that the ultrasound image is a B-mode ultrasound image.

In the ultrasound diagnostic apparatus of the present embodiment, as in the ultrasound diagnostic apparatus of the second embodiment, the operator uses the input means 60 to select the ELR transducer group scanning image 32E as one ultrasound image with the marker 72 on the display screen 71 of the display means 70. The selected ELR transducer group scanning image 32E is displayed on the display screen 71, processed by the image synthesis means 51 to be displayed relatively larger than the CLA transducer group scanning image 31E as unselected another ultrasound image. Furthermore, the signal for selecting the ELR transducer group scanning image 32E from the input means 60 is transmitted to the control means 46 as well as to the switch unit A 41 and the scan mode switch unit 42. The switch unit A 41 is switched to the ELR transducer group 32 which is the transducer group scanning the selected ELR transducer group scanning image 32E and the scan mode switch unit 42 is switched from the normal B-mode scan to Doppler-mode scan. As a result, in the selected ELR transducer group scanning image 32E, a Doppler-scan region 73 is set and a Doppler image 74 is displayed therein, as shown in FIG 15. Also, sequential switching between the B-mode scan and the Doppler-mode scan can provide an overlapped display of a Doppler-mode scan image on a B-mode scan image.

Likewise, when the CLA transducer group scanning image 31E is selected, the CLA transducer group scanning image 31E as selected one ultrasound image is displayed larger than the unselected ELR transducer group scanning image 32E. In the selected CLA transducer group scanning image 31E, the Doppler-scan region 73 is set and the Doppler image 74 is displayed therein, as shown in FIG 16.

In the ultrasound diagnostic apparatus of the present embodiment, a selection by a selection mechanism, which provides an enlarged display of one of a plurality of B-mode ultrasound images displayed, results in a simultaneous selection of the control unit of the transducer group for scanning the selected ultrasound image, to switch the scan mode from the B-mode to Doppler-mode. Thus, the ultrasound diagnostic apparatus of the present embodiment only requires the operator to select an image displaying a display part of interest with a marker on the display screen to allow relative enlargement of only an ultrasound image selected from a plurality of display images, and at the same time, an overlapped display of a selected B-mode ultrasound image and a Doppler image on the display screen 71.

The ultrasound diagnostic apparatus of the present embodiment thus provides enlarged and reduced displays of the relative display sizes of a plurality of ultrasound images simultaneously displayed. This allows effective use of the limited space of the display screen, operator selectability of an easy-to-view aspect of display, and displaying a Doppler image of an ultrasound image of operator's interest without a complicated operation, providing a good operability of the ultrasound diagnostic apparatus.

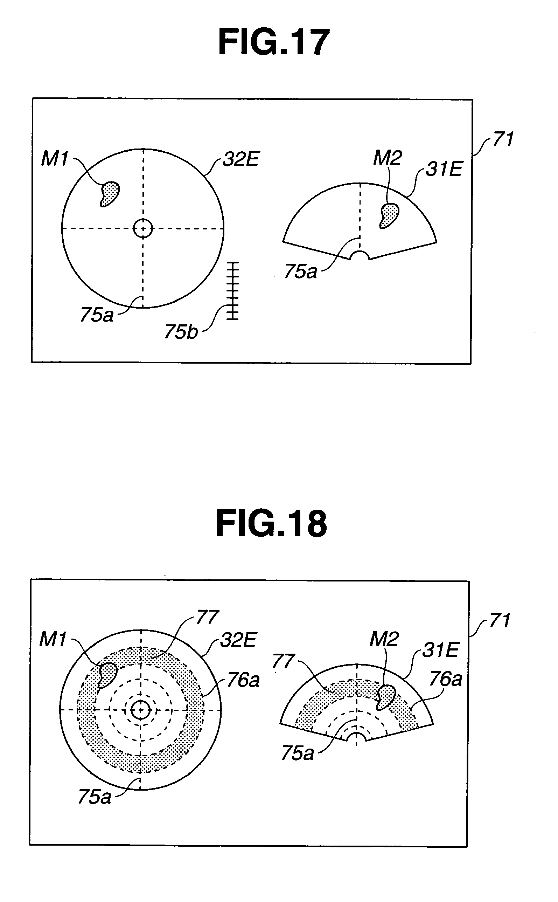

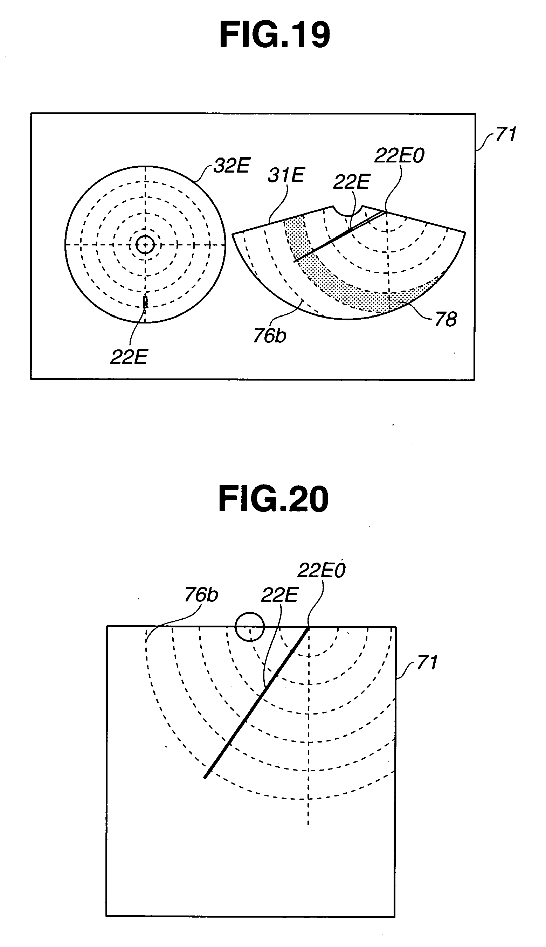

Incidentally, it is convenient to display auxiliary calibration marks that are orthogonal to the sound ray direction of each transducer on the display screen of the ultrasound diagnostic apparatus. For example, FIGS. 17 to 19 are views each showing an aspect of display with auxiliary calibration marks, simultaneously displaying two images of the CLA transducer group scanning image 31E and the ELR transducer group scanning image 32E. FIGS. 20 to 22 are each a view showing an aspect of display with auxiliary calibration marks, displaying one ultrasound image.

In FIG 17, there are displayed the CLA transducer group scanning image 31E and the ELR transducer group scanning image 32E, as well as an auxiliary calibration mark 75a and a depth-indication scale 75b to help the operator to grasp the position and invasion depth, respectively, of a lesion part M1 or M2 such as tumor. The auxiliary calibration mark 75a and the depth-indication scale 75b are both arranged in orthogonal directions with respect to the display screen 71, i.e., as ordinate and transverse axes.

In general, to measure an invasion depth of the lesion part M1 or M2 such as tumor on the ELR transducer group scanning image 32E, the distance in the radial direction is measured. While in the case of the CLA transducer group scanning image 31E, when puncturing with a biopsy needle, the distance from the biopsy needle puncture point to the lesion part M2 such as tumor as a target is generally measured. However, with the auxiliary calibration marks, etc. shown in FIG. 17, the operator cannot instantly measure such distances in the above cases. Though these measurements are made possible by a distance measurement with a measurement cursor usually provided to an ultrasound diagnostic apparatus as an auxiliary function, the operator still cannot instantly measure such distances. In particular, when conducting FNA (Fine Needle Aspiration), the following operations are repeated in the following order: (1) Displaying operation of a Doppler image for checking for bloodstream presence-absence; (2) Target distance measuring operation; (3) Needle-puncturing length adjusting operation; and (4) Puncturing operation. Accordingly, a facilitated target distance measuring operation performed in a short time period leads to a reduced procedure time period.

In the aspect of display shown in FIG 18, there are displayed the CLA transducer group scanning image 31E and the ELR transducer group scanning image 32E, as well as auxiliary calibration marks 76a in a concentric circular shape as distance measurement scales. Furthermore, a part of doughnut-shaped regions sectioned by the concentric circles of the auxiliary calibration marks 76a is provided with a colored display 77. The colored display 77 is translucent and therefore does not interfere with the scan image recognition. The doughnut-shaped regions sectioned by the concentric circles of the auxiliary calibration marks 76a may be respectively provided with differently colored displays from the inside to the outside. Though not shown, the concentric circle shaped auxiliary calibration marks 76a may be colored. Of course, auxiliary calibration marks may also be used together that are arranged in orthogonal directions to the display screen 71, i.e., as ordinate and transverse axes. The various auxiliary calibration marks and the colored display 77 can be selected by the operator to be displayed as desired as needed, or to the contrary, undisplayed.

By using the ultrasound diagnostic apparatus that displays the display screen 71 shown in FIG 18, the operator can instantly measure the distance in the radial direction from the center point of ultrasound transmission. The above auxiliary calibration marks are particularly effective in an ultrasound diagnostic apparatus having radial transducers.

FIG 19 is a view showing an aspect of display showing the CLA transducer group scanning image 31E and the ELR transducer group scanning image 32E when the biopsy needle 22 is used for puncturing in a biplane ultrasound diagnostic apparatus. Reference symbol 22E indicates a scan image of the biopsy needle 22.

Concentric circular auxiliary calibration marks 76b are also displayed centering a puncture point 22E0 of the biopsy needle 22. A partial region surrounded by the concentric circles is provided with a colored display 78. The colored display 78 is translucent and therefore does not interfere with the scan image recognition. The doughnut-shaped regions sectioned by the concentric circles of the auxiliary calibration marks 76b may be respectively provided with differently colored displays from the inside to the outside. Though not shown, the concentric circular auxiliary calibration marks 76b may each have a different color. Of course, an auxiliary calibration mark may also be used together that is arranged in orthogonal directions to the display screen 71, i.e., as ordinate and transverse axes. The auxiliary calibration marks 76b, etc. and the colored display 78 can be selected by the operator to be displayed as desired as needed, or to the contrary, undisplayed.

By using the ultrasound diagnostic apparatus that displays the concentric circular auxiliary calibration marks centering the puncture point 22E0 of the biopsy needle 22 shown in FIG 19, the operator can instantly measure a distance in the radial direction from the needle puncture point. The auxiliary calibration marks are particularly effective in an ultrasound diagnostic apparatus including convex, linear or sector transducers.

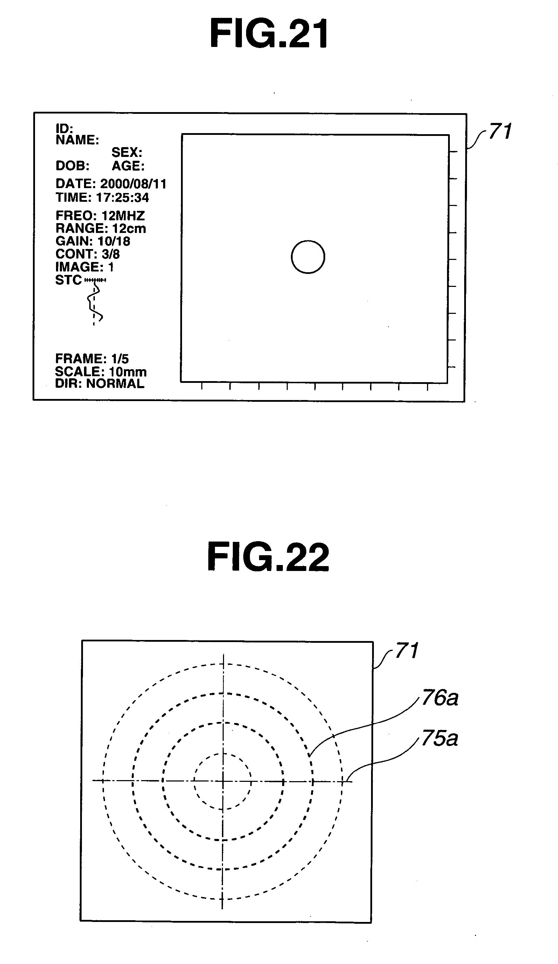

FIG 20 shows, as in FIG 19, an exemplary aspect of display displaying the concentric circular auxiliary calibration marks 76b centering the puncture point 22E0 of the biopsy needle 22. FIG 21 shows an exemplary aspect of display displaying an ultrasound image together with information on diagnosis. Auxiliary calibration marks in this aspect of display are displayed only in perpendicular and horizontal directions with respect to the screen. FIG 22 shows an exemplary aspect of display of an ultrasound diagnostic apparatus using radial ultrasonic transducers, displaying the concentric circular auxiliary calibration marks 76b and the perpendicular and horizontal auxiliary calibration marks 75a, with the probe shaft located at the center.

As indicated above, frequently used distance measurement scales are displayed as needed depending on the scanning direction of transducers used in an ultrasound diagnostic apparatus. In other words, in the case of radial transducers, scales which are concentric circular from the center of the transducers are displayed. In the case of convex, sector or linear transducers, it is preferable to display scales which are concentric circular centering the needle puncture point. In the case of biplane transducers, a desired auxiliary scale may be displayed while simultaneously displaying two images.

Having described the preferred embodiments of the invention referring to the accompanying drawings, it should be understood that the present invention is not limited to those precise embodiments and various changes and modifications thereof could be made by one skilled in the art without departing from the scope of the invention as defined in the appended claims.

## Claims

1. An ultrasound diagnostic apparatus, comprising:
a plurality of transducer groups provided to a distal end portion of a probe, for scanning in planes that are orthogonal to each other;
display means including a display screen capable of simultaneously displaying a plurality of ultrasound images obtained by the scanning by the respective transducer groups;
input means capable of selecting one of a plurality of aspects of display wherein a positional relation of the plurality of transducer groups on the probe and a positional relation of the plurality of ultrasound images on the display screen are the same; and
image synthesis means for displaying the one aspect of display selected by the input means on the display screen.

2. The ultrasound diagnostic apparatus according to Claim 1, wherein the positional relation of the plurality of transducer groups on the probe is at least one of a left-right positional relation and an up-down positional relation as observed from a viewpoint orthogonal to a scanning plane of the selected one of the plurality of transducer groups.

3. The ultrasound diagnostic apparatus according to Claim 1, wherein
the input means can select one ultrasound image on the display screen of the display means, and
the image synthesis means displays the one ultrasound image selected by the input means in an enlarged manner relative to unselected another ultrasound image on the display screen.

4. The ultrasound diagnostic apparatus according to Claim 3, further comprising an image memory for displaying on the display screen a marker with which the one ultrasound image is selectable.

5. The ultrasound diagnostic apparatus according to Claim 3, wherein scan mode of the transducer group that scans the selected one ultrasound image is switched to Doppler-mode scan.

6. The ultrasound diagnostic apparatus according to Claim 1, further comprising a commonly used sound ray synthesis table for storing control data of each of the transducer groups, wherein the plurality of transducer groups each include a plurality of transducers arranged in a circumferential or arc shape having a same radius of curvature.

7. The ultrasound diagnostic apparatus according to Claim 1, wherein ultrasound images simultaneously displayed on the display screen are ultrasound images obtained by scanning by not less than three of the transducer groups.

8. An ultrasound diagnostic apparatus, comprising:
an elongate probe;
a CLA transducer group and an ELR transducer group provided to a distal end portion of a probe, for scanning in planes that are orthogonal to each other;
display means including a display screen capable of simultaneously displaying two ultrasound images obtained by the scanning by the CLA transducer group and the ELR transducer group;
input means capable of selecting one of a plurality of aspects of display wherein a positional relation between the CLA transducer group and the ELR transducer group on the probe and a positional relation of the two ultrasound images on the display screen are the same; and
image synthesis means for displaying the one aspect of display selected by the input means on the display screen.

9. The ultrasound diagnostic apparatus according to Claim 8, wherein the positional relation between the CLA transducer group and the ELR transducer group on the probe is at least one of a left-right positional relation and an up-down positional relation as observed from a viewpoint orthogonal to a scanning plane of one of the CLA transducer group and the ELR transducer group.

10. The ultrasound diagnostic apparatus according to Claim 8, wherein
the input means can select one ultrasound image on the display screen of the display means, and
the image synthesis means displays the one ultrasound image selected by the input means in an enlarged manner relative to unselected another ultrasound image on the display screen,.

11. The ultrasound diagnostic apparatus according to Claim 10, further comprising an image memory for displaying on the display screen a marker with which the one ultrasound image is selectable.

12. The ultrasound diagnostic apparatus according to Claim 4, wherein scan mode of the transducer group that scans the selected one ultrasound image is switched to Doppler-mode scan.

13. The ultrasound diagnostic apparatus according to Claim 8, further comprising a commonly used sound ray synthesis table for storing control data of each of the CLA transducer group and the ELR transducer group,
wherein the CLA transducer group and the ELR transducer group each include a plurality of transducers arranged in a circumferential or arc shape having a same radius of curvature.
